(19) Europäisches Patentamt
European Patent Office
Office européen des brevets

(11) Veröffentlichungsnummer: **0 430 194 A2**

(12) **EUROPÄISCHE PATENTANMELDUNG**

(21) Anmeldenummer: **90122699.3**

(22) Anmeldetag: **28.11.90**

(51) Int. Cl.5: **C07D 249/08**, C07D 233/60, C07D 409/06, C07D 405/06, C07D 401/06, C07D 417/06, A61K 31/41

(30) Priorität: **30.11.89 DE 3939525**

(43) Veröffentlichungstag der Anmeldung: **05.06.91 Patentblatt 91/23**

(84) Benannte Vertragsstaaten: **AT BE CH DE FR GB IT LI NL Patentblatt**

(71) Anmelder: **HOECHST AKTIENGESELLSCHAFT**
**Postfach 80 03 20**
**W-6230 Frankfurt am Main 80(DE)**

(72) Erfinder: **Kottmann, Hariolf, Dr.**
**Am Alten Birnbaum 6**
**W-6238 Hofheim am Taunus(DE)**
Erfinder: **Blume, Ernst, Dr.**
**Rossertstrasse 20**
**W-6239 Kriftel/Taunus(DE)**
Erfinder: **Haenel, Heinz, Dr.**
**Taunusstrasse 148**
**W-6370 Oberursel(DE)**
Erfinder: **Kirsch, Reinhard, Dr.**
**Johannesallee 18**
**W-6230 Frankfurt am Main(DE)**
Erfinder: **Raether, Wolfgang, Dr.**
**Falkensteinstrasse 6**
**W-6072 Dreieich(DE)**

# (54) Mit mehrfach ungesättigten Resten substituierte Aryl-alkyl-azole, Verfahren zu ihrer Herstellung und ihre Verwendung.

(57) Aryl-alkyl-imidazole der allgemeinen Formel

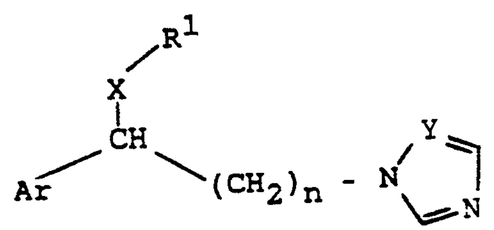

(I)

antimykotisch wirksame pharmazeutische Präparate, die solche Verbindungen enthalten, Verfahren zur Herstellung der Aryl-alkyl-imidazole, sowie Verwendung der Verbindungen zur Herstellung eines antimykotischwirksamen Arzneimittels.

EP 0 430 194 A2

# MIT MEHRFACH UNGESÄTTIGTEN RESTEN SUBSTITUIERTE ARYL-ALKYL-AZOLE, VERFAHREN ZU IHRER HERSTELLUNG UND IHRE VERWENDUNG

Die Erfindung betrifft mit mehrfach ungesättigten Resten substituierte Aryl-alkyl-azole, Verfahren zu ihrer Herstellung sowie solche Verbindungen enthaltende pharmazeutische Zusammensetzungen und ihre Verwendung als Pharmazeutika, insbesondere als Wachstumshemmer der pathogenen Phase dimorpher Hefen, als Antimykotika und Pflanzenschutzmittel, z.B. als Fungizide und Wachstumsregulatoren.

Es ist bekannt, daß Aryl-alkyl-azole unterschiedlichster Struktur als Antimykotika und Fungizide Verwendung finden. Die in der deutschen Offenlegungsschrift Nr. 20 63 857 beschriebenen 1-[ß-Aryl-ß(R-oxy)-ethyl]` imidazole zählen hierbei zu den bekanntesten Vertretern. Erfasst werden auch Verbindungen mit Alkenoxyseitenketten wie Allyloxy- und Cinnamyloxyderivate. Deren Wirkung und Verträglichkeit, insbesondere deren pilzabtötende Eigenschaften sind nicht zufriedenstellend.

Es wurde nun gefunden, daß in Abhängigkeit von der Art des in der Seitenkette vorhandenen ungesättigten Restes die Wirkung und Verträglichkeit sowie die fungiziden Eigenschaften dieser Substanzen deutlich verbessert werden konnten. Desweiteren erwiesen sich die erfindungsgemäßen Verbindungen überraschenderweise als hervorragende Wachstumshemmer der pathogenen Phase dimorpher Hefezellen.

Die Verbindung betrifft daher Aryl-alkyl-azole der Formel I

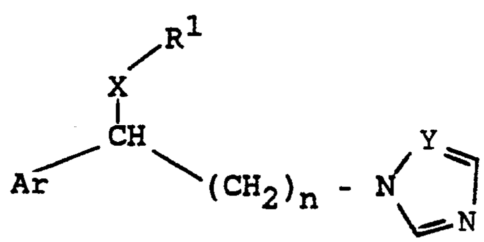

(I),

worin bedeuten

Ar Phenyl, Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Furyl, Pyridyl, Pyrimidyl, Oxazolyl, Isoxazolyl, Pyridazyl, Pyrazyl, Pyrazolyl, Benzofuryl, Benzothienyl, Benzothiazolyl, Benzoimidazolyl,

wobei die genannten Ringsysteme unsubstituiert oder mit 1 bis 3 Substituenten, die gleich oder verschieden sind, substituiert sind, welche

F, Cl, Br, I, ($C_1$-$C_8$)-Alkyl (geradkettig oder verzweigt), ($C_nF_{2n+1}$) mit n = 1-8, $C_2F_4$, Monofluorbutyl, $CCl_3$, ($C_3$-$C_8$)-Cycloalkyl, ($C_1$-$C_8$)-Alkoxy (geradkettig oder verzweigt), ($C_3$-$C_8$)-Cycloalkyl-($C_1$-$C_4$)-Alkyl, ($C_1$-$C_8$)Alkylthio, ($C_1$-$C_8$)-Alkylsulfinyl- und -sulfonyl, $NO_2$, CN, $CF_3$ sind;

Y CH oder N

n 1 oder 2

X O, S, SO, $SO_2$, $CH_2$

$R^1$ mehrfach ungesättigtes ($C_5$-$C_{20}$)-Alkenyl geradkettig oder verzweigt, mehrfach ungesättigtes ($C_5$-$C_{20}$)-Alkinyl geradkettig oder verzweigt, mehrfach ungesättigtes ($C_5$-$C_{20}$)-Alken-in-yl, geradkettig oder verzweigt,

und ihre Salze mit pharmakologisch akzeptablen Säuren.

Bevorzugt sind Verbindungen der Formel I, in denen die Substituenten und Indices folgende Bedeutung haben

Ar Phenyl, Biphenylyl, 1,2,3,4-Tetrahydronaphthyl, Naphthyl, Thienyl, Indanyl (jeweils unsubstituiert oder im aromatischen Ring mit ein oder zwei Substituenten, die gleich oder verschieden sind und jeweils F, Cl, Br, $CF_3$, ($C_1$-$C_4$)-Alkyl oder ($C_1$-$C_4$)-Alkoxy bedeuten, substituiert),

Y CH oder N

n 1 oder 2

x O, S, SO, $SO_2$, $CH_2$,

$R^1$ mehrfach ungesättigtes ($C_5$-$C_{20}$)-Alkenyl, geradkettig oder verzweigt, mehrfach ungesättigtes ($C_5$-$C_{20}$)-Alkinyl, geradkettig oder verzweigt, mehrfach ungesättigtes ($C_5$-$C_{20}$)-Alken-in-yl, geradkettig oder verzweigt,

und ihre Salze mit physiologisch verträglichen Säuren.

Besonders bevorzugt sind Verbindungen der Formel I, in denen die Substituenten und Indices folgende Bedeutung haben

Ar Phenyl, Biphenylyl, Naphthyl, Thienyl, Indanyl (jeweils unsubstituiert oder im aromatischen Ring mit ein oder zwei Substituenten, die gleich oder verschieden sind und jeweils F, Cl, $CF_3$, ($C_1$-$C_4$)-Alkyl oder ($C_1$-$C_4$)-Alkoxy bedeuten, substituiert),

Y CH oder N

n 1 oder 2

X O, S, SO, $SO_2$,

$R^1$ mehrfach ungesättigtes ($C_5$-$C_{20}$)-Alkenyl geradkettig oder verzweigt, mehrfach ungesättigtes ($C_5$-$C_{20}$)-Alkinyl geradkettig oder verzweigt, mehrfach ungesättigtes ($C_5$-$C_{20}$)-Alken-in-yl, geradkettig oder verzweigt,

und ihre Salze mit physiologisch verträglichen Säuren.

Ganz besonders bevorzugt sind Verbindungen der Formel I, in denen die Substituenten und Indices folgende Bedeutung haben

Ar Phenyl, Biphenylyl, Indanyl, Thienyl, Naphthyl, (jeweils unsubstituiert oder im aromatischen Ring mit ein oder zwei Substituenten, die gleich oder verschieden sind und F-oder Cl-Atome, $CF_3$, Methyl und Methoxy bedeuten, substituiert)

Y CH oder N

n 1

X O, S, SO, $SO_2$,

$R^1$ mehrfach ungesättigtes ($C_5$-$C_{20}$)-Alkenyl, geradkettig oder verzweigt, mehrfach ungesättigtes ($C_5$-$C_{20}$)-Alkinyl, geradkettig oder verzweigt, mehrfach ungesättigtes ($C_5$-$C_{20}$)-Alken-in-yl, geradkettig oder verzweigt,

und ihre Salze mit physiologisch verträglichen Säuren.

Die als Substituenten oder in Zusammenhang mit anderen Substituenten auftretenden ($C_1$-$C_8$)- bzw. ($C_1$-$C_4$)-Alkylgruppen können unverzweigt oder verzweigt sein und beispielsweise die Methyl-, Ethyl-, Propyl-, 1-Methylethyl-, Butyl-, 2-Methylpropyl-, 1,1-Dimethylethyl-, Pentyl-, Hexyl-, Heptyl-, Oktyl-, Nonylgruppe bedeuten;

durch Fluor- oder Chlor substituierte Alkylgruppen können beispielsweise die Trifluormethyl-, Trichlormethyl-, 1,1,2,2-Tetrafluorethyl oder die Nonafluorbutylgruppe bedeuten;

die ($C_3$-$C_8$)-Cycloalkylgruppen können die Cyclopropyl-, Cyclobutyl-, Cyclopentyl-, Cyclohexyl-, Cycloheptyl-, oder Cyclooctylgruppe bedeuten;

die ($C_3$-$C_8$)-Cycloalkyl-($C_1$-$C_4$)-alkylgruppen können beispielsweise die Cyclopropylmethyl-, Cyclopentylmethyl-, Cyclohexylmethyl-, Cyclohexylethyl-, Cycloheptylmethyloder Cyclooctylmethyl-Gruppe bedeuten.

Die mehrfach ungesättigten ($C_5$-$C_{20}$)-Alkenyl-, Alkinyl-, Alken-in-yl-reste können nicht konjugierte und/oder konjugierte Mehrfachbindungen aufweisen, wobei die Anzahl der Mehrfachbindungen 2 bis 4 betragen kann, sowie die Verbindungen bezüglich der Konfiguration der Doppelbindungen als reine cis- oder als reine trans-Isomere, aber auch als cis/trans-Isomerengemische vorliegen können, beispielsweise 4-Methyl-2,4-pentadien-1-yl, 2-Methyl-2,4-pentadien-1-yl, trans-trans-2,4-Hexadien-1-yl, 1,5-Hexadien-3-yl, 3,5-Hexadien-1-yl, 2,5-Hexadien-1-yl, 2,6-Heptadien-1-yl, 2,4-Dimethyl-2,6-heptadien-1-yl, 1,6-Heptadien-4-yl, 1,7-Octadien-4-yl, cis-3,7-Dimethyl-2,6-octadien-1-yl, trans-3,7-Dimethyl-2,6-octadien-1-yl, trans-cis-3,7-Dimethyl-2,4,6-octatrien-1-yl, trans-trans-2,6-Dimethyl-2,4,6-octatrien-1-yl, 1,8-Nonadien-4-yl, 3,7,11-Trimethyl-2,6,10-dodecatrien-1-yl, 3,7,11-Trimethyl-1,6,10-dodecatrien-3-yl.

Die Erfindung betrifft die Verbindungen I in Form der freien Base oder eines Säureadditionssalzes.

Beispiele pharmazeutisch akzeptabler salzbildender Säuren sind anorganische Säuren wie Salzsäure,

Bromwasserstoffsäure, Jodwasserstoffsäure, Schwefelsäure, Phosphorsäure oder Salpetersäure oder organische Säuren wie Malonsäure, Oxalsäure, Gluconsäure, Camphersulfonsäure, Benzolsulfonsäure, Essigsäure, Propionsäure, p-Toluolsulfonsäure oder Salicylsäure.

Die Verbindungen I weisen mindestens ein asymmetrisches C-Atom auf und können daher als Enantiomere und Diasteromere auftreten. Die Erfindung umfaßt sowohl die reinen Isomeren als auch deren Gemische. Die Gemische von Diastereomeren können nach gebräuchlichen Methoden, z.B. durch selektive Kristallisation aus geeigneten Lösungsmitteln oder durch Chromatographie an Kieselgel oder Aluminiumoxid in die Komponenten aufgetrennt werden.

Racemate können nach üblichen Methoden in die Enantiomeren aufgetrennt werden, so z.B. durch Salzbildung mit einer optisch aktiven Säure, Trennung der diastereomeren Salze und Freisetzung der reinen Enantiomeren mittels einer Base. Darüber hinaus lassen sich die Enantiomeren auch mittels Chromatographie an chiralen Phasen oder auf enzymatischem Wege trennen.

Die Erfindung betrifft weiterhin ein Verfahren zur Herstellung von Verbindungen der Formel (I) dadurch gekennzeichnet, daß man eine Verbindung der Formel II

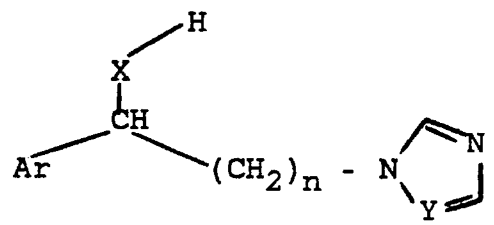

(II),

in welcher Ar, X, n und Y die in Anspruch 1 angegebenen Bedeutungen haben,

mit einer Verbindung der Formel (III)

$$R^1 - Nu \qquad (III)$$

umsetzt, worin $R^1$ die unter Anspruch 1 angegebene Bedeutung hat und Nu Fluor, Chlor, Brom, Jod, Trifluormethansulfonat, p-Toluolsulfonat, Methansulfonat oder ein anderes üblicherweise verwendetes Nucleofug bedeutet,

und eine so erhaltene Verbindung der Formel (I) in Form der freien Base oder eines Säureadditionsalzes oder eines hydrolysierbaren Derivats isoliert.

Zur Herstellung der Verbindungen der Formel I wird eine Verbindung der Formel II mit einer Verbindung der Formel III in einem inerten Lösungsmittel gegebenenfalls in Gegenwart einer Base innerhalb eines Temperaturbereichs von 0° C bis 150° C, vorzugsweise zwischen 20° C und 120° C umgesetzt.

Geeignete Lösungsmittel sind Acetonitril, N,N-Dimethylformamid, N,N-Dimethylacetamid, Dimethylsulfoxid, Sulfolan, N-Methylpyrrolidon-2, Dioxan oder Tetrahydrofuran. Es können auch Gemische dieser beispielhaft genannten Lösungsmittel verwendet werden.

Geeignete Basen sind beispielsweise Alkalimetall- oder Erdalkalimetall-carbonate, -hydrogencarbonate, -hydroxide, -alkoholate oder -hydride, z.B. Natriumcarbonat, Natriumhydrogencarbonat, Kaliumcarbonat, Natriumhydroxid, Natriumethylat oder Natriumhydrid oder organische Basen z.B. tertiäre Amine wie Triethylamin, Tributylamin, Ethylmorpholin oder Pyridin, Dimethylaminopyridin, Chinolin oder 1,5-Diazabicyclo-[5,4,0]-undec-5-en-(1,8,7) (DBU), 1-H-Imidazol oder 1H-1,2,4-Triazol.

Verbindungen der Formel III, in denen Nu = Fluor, Chlor, Brom, Jod, Trifluormethansulfonat, p-Toluolmethansulfonat, Methansulfonat oder ein üblicherweise verwendetes Nucleofug bedeutet, werden aus den entsprechenden Vorstufen nach literaturbekannten Verfahren hergestellt, in dem z.B. der entsprechende Alkohol (Nu = OH) in Gegenwart einer Base, z.B. Pyridin, mit Säurehalogeniden wie Thionylchlorid oder Oxalylchlorid in einem inerten Verdünnungsmittel oder ohne Lösungsmittel in einem Temperaturbereich von -30° C bis 60° C umgesetzt wird.

Verbindungen der Formel II, in denen X = O und Ar, n und Y die in Anspruch 1 angegebene Bedeutung haben, werden nach literaturbekannten Syntheseverfahren hergestellt z.B. nach einem Verfahren, in dessen Verlauf ein Keton der Formel IV

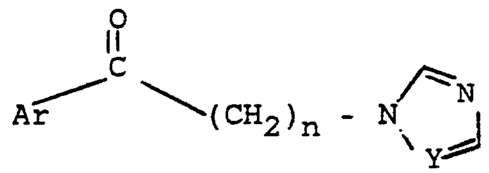 (IV)

mit üblichen Reduktionsmitteln, wie $NaBH_4$ zur gewünschten Verbindung umgesetzt wird.

Verbindungen der Formel I, in denen Ar, X, n und Y die unter Anspruch 1 angegebene Bedeutung haben, können außerdem durch Umsetzung von Verbindungen der Formel V

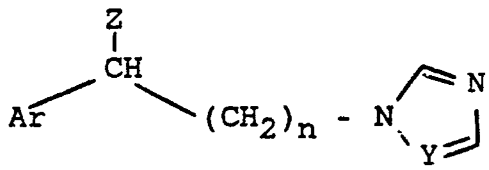 (V),

in denen Z Fluor, Chlor, Brom, Jod, Trifluormethansulfonat, p-Toluolmethansulfonat, Methansulfonat oder ein anderes üblicherweise verwendetes Nucleofug bedeutet,

mit Verbindungen der Formel VI

$$R^1 - X\,H \qquad (VI)$$

in denen $R^1$ und X die in Anspruch 1 angegebene Bedeutung haben, erhalten werden.

Reaktionen dieser Art finden bevorzugt in einem inerten Lösungsmittel gegebenenfalls in Gegenwart einer Base innerhalb eines Temperaturbereichs von -10° C bis 150° C, vorzugsweise 20° C bis 100° C statt.

Als geeignete Lösungsmittel und als geeignete Basen kommen die oben genannten Lösungsmittel und Basen in Betracht.

Die Erfindung betrifft weiterhin ein Verfahren zur Herstellung von Verbindungen der Formel I dadurch gekennzeichnet, daß man eine Verbindung der Formel V, in welcher Z Fluor, Chlor, Brom, Jod, Trifluormethansulfonat, p-Toluolmethansulfonat, Methansulfonat oder ein anderes üblicher Weise verwendetes Nucleofug bedeutet,

mit einer Verbindung der Formel VI, worin $R^1$ und X die in Anspruch 1 angegebene Bedeutung haben, unter den Bedingungen einer Phasentransferreaktion umsetzt.

Dazu wird eine Lösung der Verbindung der Formel V in einem inerten Lösungsmittel unter kräftigem Rühren, in Anwesenheit eines Phasentransferkatalysators und einer Base, vorzugsweise einem gepulverten Alkalihydroxid oder -carbonat, z.B. Natrium- oder Kaliumhydroxid oder Carbonat oder einer konzentrierten wäßrigen Lösung derselben, vorzugsweise in einem Temperaturbereich von 20 - 100° C, mit einer Verbindung der Formel VI umgesetzt.

Geeignete Lösungsmittel sind beispielsweise, N,N-Dimethylformamid, N,N-Dimethylacetamid, Dimethylsulfoxid, Sulfolan, N-Methylpyrrolidon, Dioxan, Tetrahydrofuran, Acetonitril, 4-Methyl-2-pentanon, Methanol, Ethanol, Isopropanol, Propanol, Butanol, Pentanol, Tert-butylalkohol, Methylglykol, Eisessig,

Methylenchlorid, Chloroform, Tetrachlorkohlenstoff oder ein aromatischer Kohlenwasserstoff wie Benzol, Chlorbenzol, Nitrobenzol, Toluol oder Xylol oder Wasser. Es können auch Gemische der beispielhaft genannten Lösungsmittel verwendet werden.

Geeignete Phasentransferkatalysatoren sind guaternäre Ammonium- oder Phosphoniumsalze sowie Kronenether oder Polyethylenglykole. Als besonders geeignet für die vorliegenden Zwecke erwiesen sich Tetrabutylammoniumbromid und Butyltriethylammoniumchlorid.

Bei diesen unter Phasentransferbedingungen ablaufenden Umsetzungen können bei Verwendung von Verbindungen der Formel VI, in welchen $R^1$ die in Anspruch 1 genannte Bedeutung hat und X = 5 bedeutet, auch Derivate oder Vorstufen dieser Verbindungen wie z.B. Thiuroniumsalze der Formel VII

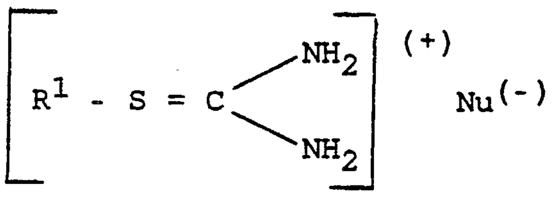

(VII)

eingesetzt werden, wobei $R^1$ und Nu die oben genannten Bedeutung haben.

Verbindungen der Formel VII sind überwiegend noch nicht in der Literatur beschrieben und werden entsprechend literaturbekannten Verfahren hergestellt, wie z.B. durch Umsetzung von Thioharnstoff mit einer Verbindung der Formel III, wobei $R^1$ und Nu die oben genannte Bedeutung haben, in einem Lösungsmittel z.B. Ethanol in einem Temperaturbereich von 40 °C - 120 °C.

Verbindungen der Formel VI sind überwiegend noch nicht bekannt und in der Literatur noch nicht beschrieben. Sie werden nach literaturbekannten Verfahren, die allgemein zur Synthese von Mercaptanen und Alkoholen angewandt werden, hergestellt.

Verbindungen der Formel V, in denen Z = F, Cl oder Br bedeutet, können hergestellt werden, indem man eine Verbindung der Formel V mit Z = OH mit Thionylchlorid, Schwefeltetrafluorid oder Diethylaminoschwefeltrifluorid umsetzt, in einem inerten Lösungsmittel, gegebenenfalls in Gegenwart einer Base, vorzugsweise in einem Temperaturbereich von 0 °C - 80 °C.

Verbindungen der Formel I, die eine Thioethergruppe enthalten, können am Schwefel zu einem Sulfoxid oder Sulfon oxidiert werden. Dazu werden solche Verbindungen der Formel I mit einem Oxidationsmittel, z.B. Wasserstoffperoxid oder m-Chlorperbenzoesäure umgesetzt, in einem inerten Lösungsmittel, in einem Temperaturbereich von -10 °C bis 80 °C. Zur Herstellung von Sulfoxiden setzt man dazu ein Moläquivalent Oxidationsmittel ein, zur Herstellung von Sulfonen zwei Moläquivalente, gegebenenfalls auch einen Überschuß.

Als Lösungsmittel werden bevorzugt die oben genannten verwendet.

Die Verbindungen der Formel I, ihre Säureadditionssalze und ihre physiologisch hydrolysierbaren Derivate sind wertvolle Arzneimittel. Sie wirken insbesondere antimikrobiell und eignen sich zur Vorbeugung und Behandlung von Pilzinfektionen beim Menschen und bei verschiedenen Säugetierarten.

Die neuen Verbindungen sind in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen grampositive und gramnegative Bakterien.

Auch in vivo, z.B. bei der experimentellen Nierencandidose der Maus, besitzen die Verbindungen nach oraler oder parenteraler Anwendung einen sehr guten systemischen Effekt, z.B. gegen Candida albicans. Hierbei wird von der Hefe Candida albicans insbesondere das Exoenzymsystem dergestalt beeinflußt, daß die Pathogenität der Erreger deutlich absinkt. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Als Indikationsgebiete in der Humanmedizin können beispielsweise genannt werden:

Dermatomykosen und Systemmykosen durch Trichophyton mentagrophytes und andere Trichophytonarten, Mikrosporenarten, Epidermophyton floccosum, und biphasische Pilze sowie Schimmelpilze hervorgerufen. Insbesondere werden tiefe Mykosen, die durch Candida albicans hervorgerufen werden, günstig beeinflußt, da hierbei ein Eindringen der Pilze in die Wirtszelle verhindert wird.

Als Indikationsgebiete in der Tiermedizin können beispielsweise aufgeführt werden:

Alle Dermatomykosen und Systemmykosen, insbesondere solche, die durch die oben genannten Erreger hervorgerufen werden.

Zur vorliegenden Erfindung gehören phamazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

Die therapeutisch wirksamen Verbindungen sollen in den oben aufgeführten pharmazeutischen Zubereitungen vorzugsweise in einer Konzentration von etwa 0,1 bis 99,5 , vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von mindestens etwa 0,05, vorzugsweise 0,1 , insbesondere 0,5 mg/kg Körpergewicht bis höchstens etwa 200, vorzugsweise bis 100, insbesondere bis 10 mg/kg Körpergewicht je 24 Stunden, gegebenenfalls in Form mehrerer Einzelgaben zur Erzielung der gewünschten Ergebnisse zu verabreichen. Die Gesamtmenge wird in 1 bis 8, vorzugsweise in 1 bis 3 Einzeldosen, bei tiefen Mykosen jedoch über wesentlich längere Zeiträume (bis zu 6 Wochen) verabreicht.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen der Formel (I) sind auch als Biozide wirksam, vorzugsweise die Triazolverbindungen, in denen Y Stickstoff bedeutet. Sie zeichnen sich insbesondere durch ihre fungizide Wirksamkeit bei phytopathogenen Pilzen aus. Selbst bereits in das pflanzliche Gewebe eingedrungene pilzliche Krankheitserreger lassen sich erfolgreich bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der neuen Verbindungen erfaßt eine Vielzahl verschiedener phytopathogener Pilze, wie z.B. Piricularia oryzae, Plasmopara viticola, verschiedene Rostarten, vor allem aber Venturia inaequalis, Cercospora beticola und echte Mehltaupilze im Obst-, Gemüse-, Getreide- und Zierpflanzenbau.

Die neuen Verbindungen der allgemeinen Formel (I) eignen sich ferner für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die neuen Verbindungen können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Unter Spritzpulvern werden in Wasser gleichmäßig dispergierbare Präparate verstanden, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel z.B. ligninsulfonsaures Natrium, 2,2'-dinaphthylmethan-6,6'-di-sulfonsaures Natrium, dibutylnaphthalinsulfonsaures Natrium oder auch oleoylmethyltaurinsaures Natrium enthalten. Ihre Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: alkylarylsulfonsaure Calciumsalze, wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel werden durch Vermahlen des Wirkstoffes mit fein verteilten, festen Stoffen, z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinit oder von granuliertem Inertmaterial. Auch können geeignete Wirkstoffe in der für die Herstellung von Düngemittelgranulaten üblichen Weise - gewünschtenfalls in Mischung mit Düngemitteln, granuliert werden.

In Spritzpulvern beträgt die Wirkstoffkonzentration z.B. etwa 10-90 Gew.-%, der Rest zu 100 Gew.-% besteht aus üb-lichen Formulierungsbestandteilen. Bei emulgierbaren

Konzentraten kann die Wirkstoffkonzentration etwa 10-80 Gew.-% an Wirkstoff, bei versprühbaren Lösungen etwa 1-20 Gew.-% betragen. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z.B. Insektiziden, Akariziden, Herbiziden, Düngemitteln, Wachstumsregulatoren oder weiteren Fungiziden sind gegebenenfalls möglich, wobei u.U. auch synergistische Wirkungssteigerungen erzielt werden können.

Im folgenden seien einige Formulierungsbeispiele angeführt:

Ein Stäubemittel wird erhalten, indem man 10 Gewichtsteile Wirkstoff und 90 Gewichtsteile Talkum als Inertstoff mischt und in einer Schlagmühle zerkleinert.

Ein in Wasser leicht dispergierbares, benetzbares Pulver wird erhalten, indem man 25 Gewichtsteile Wirkstoff, 65 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat stellt man her, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z.B. ca. 255 bis über 377° C) mischt und in einer Reibkugelmühle auf eine Feinheit von unter 5 Mikron vermahlt.

Ein emulgierbaren Konzentrat läßt sich herstellen aus 15 Gewichtsteilen Wirkstoff, 75 Gewichtsteilen Cyclohexanon als Lösungsmittel und 10 Gewichtsteilen oxethyliertem Nonylphenol (10 AeO) als Emulgator.

Die nachfolgenden Beispiele dienen zur näheren Erläuterung der Erfindung.

Beispiel 1 (Vorstufe)

cis-3.7-Dimethyl-2,6-octadien-1-chlorid

Zu einer Lösung von 46,27 g (300 mMol) cis-3,7-Dimethyl-2,6-octadien-1-ol in 600 ml n-Pentan werden unter heftigem Rühren bei -5° C 34,30 g (300 mMol) Methansulfochlorid zugetropft. Danach werden zu dieser Lösung 47,40 g (600 mMol) Pyridin gegeben. Man läßt das Reaktionsgemisch unter Rühren im Eisbad langsam auf 25° C erwärmen.

Nach Abtrennen der Pentanphase wird diese mit 5 %iger kalter Salzsäure und anschließend mit wäßriger Sodalösung gewaschen. Die organische Phase wird über $MgSO_4$ getrocknet, eingeengt und anschließend fraktioniert destilliert. Es werden 30.9 g (60 % d. Th.) an Endprodukt erhalten (Kp. 49° C/0.6 Torr).

Beispiel 2

(R,S)-1-(2,4-Dichlorphenyl)-1-(cis-3,7-dimethyl-2,6-octadien-1-oxy)-2-(imidazol-1-yl)-ethan

7,7 g (30 mMol) (R,S)-1-(2,4-Dichlorphenyl)-1-hydroxy-2-(imidazol-1-yl)-ethan werden unter Rühren in 40 ml DMF gelöst. Hierzu gibt man 1,0 g (33 mMol) Natriumhydrid in kleinen Portionen und rührt bis zum Ende der $H_2$-Entwicklung. Anschließend tropft man unter Rühren 5,7 g (33 mMol) cis-3,7-Dimethyl-2,6-octadien-1-chlorid, gelöst in 10 ml DMF, zu. Nach 12 h Reaktionszeit wird DMF im Hochvakuum abgezogen, der verbleibende Rückstand in 50 ml Methylenchlorid aufgenommen, 3mal mit $H_2O$ gewaschen, über $MgSO_4$ getrocknet und eingeengt.

Nach Chromatographie mit Methanol/Essigsäureethylester (1/10) über Kieselgel erhält man 9,2 g (78 % d. Th.) eines leicht viskosen Öls.

Beispiel 3

(R,S)-1-(4-Chlorphenyl)-1-(cis-3,7-dimethyl-2,6-octadien-1-thio)-2-(imidazol-1-yl)-ethan

2,0 g (R,S)-1-(4-Chlorphenyl)-1-(methylsulfonyl)-2-(imidazol-1-yl)-ethan werden in 50 ml Toluol gelöst und mit 1,65 g cis-3,7-Dimethyl-2,6-octadien-1-isothiuroniumchlorid sowie 5,3 g 50 %iger wäßriger Natronlauge und 0,25 g Tetrabutylammoniumbromid bei 0 °C versetzt und unter heftigem Rühren innerhalb von 2 h zur Reaktion gebracht. Anschließend wird mit $CH_2Cl_2$ extrahiert, die organische Phase mit wäßriger Sodalösung neutral gewaschen, über $MgSO_4$ getrocknet und eingeengt. Nach Chromatographie mit Essigsäureethylester über Kieselgel erhält man 0,85 g (34 % d. Th.) eines viskosen Öls.

Die folgenden in Tabelle 1 aufgeführten Verbindungen werden entsprechend den Vorschriften 1 - 3 hergestellt:

**Tabelle 1**

| Beisp. Nr. | Ar | X | $R^1$ | Y | n | Fp. (°C) |
|---|---|---|---|---|---|---|
| 4 | 2,4-$C_6H_3Cl_2$ | O | cis-3,7-dimethyl-2,6-octadien-1-yl | CH·p-TsOH | 1 | 109-111 |
| 5 | " | O | " | N | 1 | a) |
| 6 | " | O | " | CH | 2 | a) |
| 7 | " | O | " | N | 2 | a) |
| 8 | " | S | " | CH | 1 | a) |
| 9 | " | S | " | CH·p-TsOH | 1 | 96-97 |
| 10 | " | SO | " | CH | 1 | a) |
| 11 | " | $SO_2$ | " | CH | 1 | a) |
| 12 | " | $SO_2$ | " | CH·p-TsOH | 1 | 80-85 |
| 13 | " | S | " | N | 1 | a) |
| 14 | " | O | trans-3,7-dimethyl-2,6-octadien-1-yl | CH | 1 | a) |
| 15 | " | O | " | N | 1 | a) |
| 16 | " | O | " | CH·p-TsOH | 1 | 95-97 |

**Fortsetzung Tabelle 1**

| Beisp. Nr. | Ar | X | $R^1$ | Y | n | Fp. (°C) |
|---|---|---|---|---|---|---|
| 17 | " | O | 3,7,11-Trimethyl-2,6,10-dodecatrien-1-yl | CH | 1 | a) |
| 18 | " | O | " | N | 1 | a) |
| 19 | " | O | 2,4-Dimethyl-2,6-heptadien-1-yl | CH | 1 | a) |
| 20 | " | O | 1,5-Hexadien-3-yl | CH | 1 | a) |
| 21 | " | O | 2,6-Heptadien-1-yl | CH | 1 | a) |
| 22 | 2,4-$C_6H_3Cl_2$ | O | 2-Methyl-2,4-pentadien-1-yl | CH | 1 | a) |
| 23 | 4-$C_6H_4Cl$ | O | cis-3,7-Dimethyl-2,6-octadien-1-yl | CH | 1 | a) |
| 24 | " | O | " | N | 1 | a) |
| 25 | " | O | trans-3,7-dimethyl-2,6-octadien-1-yl | CH | 1 | a) |
| 26 | " | O | " | N | 1 | a) |
| 27 | " | O | " | CH·p-TsOH | 1 | 153-155 |

**Fortsetzung Tabelle 1**

| Beisp. Nr. | Ar | X | $R^1$ | Y | n | Fp. (°C) |
|---|---|---|---|---|---|---|
| 28 | " | $SO_2$ | cis-3,7-dimethyl-2,6-octadien-1-yl | CH | 1 | a) |
| 29 | " | $SO_2$ | " | CH- ·p-TsOH | 1 | 96-97 |
| 30 | " | S | 3,7,11-Trimethyl-2,6,10-dodecatrien-1-yl | CH | 1 | a) |
| 31 | 4-$C_6H_4F$ | O | cis-3,7-dimethyl-2,6-octadien-1-yl | CH | 1 | a) |
| 32 | " | O | trans-3,7-dimethyl-2,6-octadien-1-yl | CH | 1 | a) |
| 33 | 2,4-$C_6H_3F_2$ | O | cis-3,7-dimethyl-2,6-octadien-1-yl | CH | 1 | a) |
| 34 | 3-$C_6H_4F$ | O | " | CH | 1 | a) |
| 35 | Biphenyl-1-yl | O | " | CH | 1 | a) |
| 36 | Naphth-1-yl | O | cis-3,7-dimethyl-2,6-octadien-1-yl | CH | 1 | a) |
| 37 | Thien-2-yl | O | " | CH | 1 | a) |
| 38 | " | O | " | N | 1 | a) |
| 39 | " | S | " | N | 1 | a) |
| 40 | " | O | " | CH | 2 | a) |
| 41 | 5-Chlor-thien-2-yl | O | " | CH | 1 | a) |

**Fortsetzung Tabelle 1**

| Beisp. Nr. | Ar | X | $R^1$ | Y | n | Fp. (°C) |
|---|---|---|---|---|---|---|
| 42 | 5-Chlor-thien-2-yl | O | " | N | 1 | a) |
| 43 | " | O | trans-3,7-dimethyl-2,6-octadien-1-yl | CH | 1 | a) |
| 44 | " | O | " | N | 1 | a) |
| 45 | " | O | 3,7,11-Trimethyl-2,6,10-dodecatrien-1-yl | CH·p-TsOH | 1 | 128-130 |
| 46 | Pyrid-2-yl | O | cis-3,7-dimethyl-2,6-octadien-1-yl | CH | 1 | a) |
| 47 | Furan-2-yl | O | " | CH | 1 | a) |
| 48 | Thiazol-2-yl | O | " | CH | 1 | a) |

Erläuterungen zu Tabelle 1

a) die Charakterisierung der Substanzen erfolgte über Analyse und Spektroskopie. Ein genauer Festpunkt kann nicht angegeben werden. Es handelt sich um wachsartige Öle verschiedenster Viskosität, die auch nach längerer Zeit keine Tendenz zur Kristallisation zeigen.

In Tabelle 2 sind die Elementaranalysen der in Tabelle 1 aufgeführten Beispiele zusammengestellt.

**Tabelle 2** Elementaranalysen der in Tabelle 1 aufgeführten Verbindungen

| Beispiel Nr. | Summenformel | Molmasse | Elementaranalyse berechnet/gefunden | | | | | | | | | | |
|---|---|---|---|---|---|---|---|---|---|---|---|---|---|
| | | | C | | H | | N | | Cl | | F | S | |
| 1 | $C_{10}H_{17}Cl$ | 172,69 | 69,54 | 69,50 | 9,92 | 9,98 | | | 20,52 | 20,35 | | | |
| 2 | $C_{21}H_{26}Cl_2N_2O$ | 393,35 | 64,12 | 64,21 | 6,66 | 6,62 | 7,12 | 7,10 | 18,02 | | | | |
| 3 | $C_{21}H_{27}ClN_2S$ | 374,97 | 67,26 | 67,19 | 7,25 | 7,31 | 7,47 | 7,52 | 9,46 | | | 18,02 | |
| 4 | | | | | | | | | | | | | |
| 5 | $C_{20}H_{25}Cl_2N_3O$ | 394,34 | 60,91 | 60,88 | 6,39 | 6,36 | 10,65 | 10,70 | 17,99 | | | | |
| 6 | $C_{22}H_{28}Cl_2N_2O$ | 407,38 | 64,86 | 64,81 | 6,92 | 6,99 | 6,87 | 6,81 | 17,42 | | | | |
| 7 | $C_{21}H_{27}Cl_2N_3O$ | 408,37 | 61,76 | 61,77 | 6,66 | 6,61 | 10,29 | 10,35 | 17,37 | 17,40 | | | |
| 8 | $C_{21}H_{26}Cl_2N_2S$ | 409,42 | 61,60 | 61,53 | 6,40 | 6,48 | 6,84 | 6,80 | | | | 25,16 | |
| 9 | | | | | | | | | | | | | |

**Fortsetzung Tabelle 2**

| Beispiel Nr. | Summenformel | Molmasse | Elementaranalyse berechnet/gefunden C | | H | | N | | Cl | | F | | S | |
|---|---|---|---|---|---|---|---|---|---|---|---|---|---|---|
| 10 | $C_{21}H_{26}Cl_2N_2SO$ | 425,42 | 59,29 | 59,35 | 6,16 | 6,09 | 6,58 | 6,64 | 16,66 | | | | 7,53 | |
| 11 | $C_{21}H_{26}Cl_2N_2SO_2$ | 441,42 | 57,14 | 57,10 | 5,93 | 5,98 | 6,34 | 6,42 | 16,06 | | | | 7,63 | |
| 12 | $C_{28}H_{34}Cl_2N_2O_5S_2$ | 613,62 | 54,81 | 54,75 | 5,59 | 5,70 | 4,57 | 4,66 | 11,56 | 11,48 | | | | |
| 13 | $C_{20}H_{25}Cl_2N_3S$ | 410,41 | 58,53 | 58,59 | 6,14 | 6,22 | 10,24 | 10,30 | 17,27 | | | | 7,81 | |
| 14 | $C_{21}H_{26}Cl_2N_2O$ | 393,35 | 64,12 | 64,18 | 6,66 | 6,71 | 7,12 | 7,06 | 18,02 | | | | | |
| 15 | $C_{26}H_{25}Cl_2N_3O$ | 394,34 | 60,91 | 60,97 | 6,39 | 6,45 | 10,65 | 10,61 | 17,99 | 18,10 | | | | |
| 16 | $C_{18}H_{34}Cl_2N_2O_3S$ | 549,56 | 61,19 | 61,11 | 6,23 | 6,18 | 5,09 | 5,13 | 12,90 | | | | 5,83 | |
| 17 | $C_{26}H_{34}Cl_2N_2O$ | 461,47 | 67,67 | 67,69 | 7,42 | 7,29 | 6,07 | 6,02 | 15,36 | | | | | |
| 18 | $C_{25}H_{33}Cl_2N_3O$ | 462,46 | 64,93 | 64,87 | 7,19 | 7,24 | 9,08 | 9,14 | 15,33 | 15,1 | | | | |

**Fortsetzung Tabelle 2**

| Beispiel Nr. | Summenformel | Molmasse | Elementaranalyse berechnet/gefunden C | | H | | N | | Cl | | F | | S | |
|---|---|---|---|---|---|---|---|---|---|---|---|---|---|---|
| 19 | $C_{20}H_{24}Cl_2N_2O$ | 379,32 | 63,30 | 63,35 | 6,37 | 6,39 | 7,38 | 7,36 | 18,69 | | | | | |
| 20 | $C_{17}H_{18}Cl_2N_2O$ | 337,25 | 60,54 | 60,48 | 5,38 | 5,31 | 8,30 | 8,35 | 21,02 | 21,10 | | | | |
| 21 | $C_{18}H_{20}Cl_2N_2O$ | 351,27 | 61,54 | 61,59 | 5,74 | 5,80 | 7,97 | 8,02 | 20,16 | | | | | |
| 22 | $C_{17}H_{18}Cl_2N_2O$ | 337,25 | 60,54 | 60,10 | 5,38 | 5,43 | 8,30 | 8,27 | 21,02 | 20,95 | | | | |
| 23 | $C_{21}H_{27}ClN_2O$ | 358,910 | 70,28 | 70,21 | 7,58 | 7,55 | 7,81 | 7,84 | 9,88 | | | | | |
| 24 | $C_{20}H_{26}ClN_3O$ | 359,890 | 66,75 | 66,70 | 7,28 | 7,31 | 11,68 | 11,70 | 9,85 | | | | | |
| 25 | $C_{21}H_{27}ClN_2O$ | 358,910 | 70,28 | 70,33 | 7,58 | 7,62 | 7,81 | 7,78 | 9,88 | | | | | |
| 26 | $C_{20}H_{26}ClN_3O$ | 359,890 | 66,75 | 66,20 | 7,28 | 7,20 | 11,68 | 11,72 | 9,85 | | | | | |
| 27 | $C_{28}H_{35}ClN_2O_4S$ | 531,114 | 63,32 | 63,41 | 6,64 | 6,69 | 5,28 | 5,31 | 6,68 | | | | 6,04 | |

**Fortsetzung Tabelle 2**

| Beispiel Nr. | Summenformel | Molmasse | Elementaranalyse berechnet/gefunden C | | H | | N | | Cl | | F | S | |
|---|---|---|---|---|---|---|---|---|---|---|---|---|---|
| 28 | $C_{21}H_{27}ClN_2O_2$ | 406,975 | 61,98 | 61,88 | 6,69 | 6,74 | 6,88 | 7,92 | 8,71 | | | 7,88 | |
| 29 | $C_{28}H_{35}ClN_2O_5S_2$ | 579,179 | 58,07 | 58,12 | 6,09 | 6,02 | 4,84 | 4,80 | 6,12 | | | 11,07 | |
| 30 | $C_{26}H_{35}ClN_2S$ | 443,096 | 70,48 | 70,42 | 7,96 | 7,89 | 6,32 | 6,28 | 8,00 | 8,11 | | 7,24 | |
| 31 | $C_{21}H_{27}FN_2O$ | 342,55 | 73,65 | 73,60 | 7,95 | 7,88 | 8,18 | 8,09 | | | 5,55 | | |
| 32 | $C_{21}H_{27}FN_2O$ | 342,455 | 73,65 | 73,68 | 7,95 | 7,87 | 8,18 | 8,24 | | | 5,55 | | |
| 33 | $C_{21}H_{26}F_2N_2O$ | 360,445 | 69,98 | 69,90 | 7,27 | 7,33 | 7,77 | 7,81 | | | 10,54 | | |
| 34 | $C_{21}H_{27}FN_2O$ | 342,455 | 73,65 | 73,69 | 7,95 | 8,04 | 8,18 | 8,23 | | | 5,55 | | |
| 35 | $C_{27}H_{32}N_2O$ | 400,563 | 80,96 | 80,89 | 8,05 | 7,99 | 6,99 | 7,04 | | | | | |
| 36 | $C_{25}H_{30}N_2O$ | 374,525 | 80,175 | 80,14 | 8,07 | 8,13 | 7,48 | 7,39 | | | | | |

**Fortsetzung Tabelle 2**

| Beispiel Nr. | Summenformel | Molmasse | Elementaranalyse berechnet/gefunden C | | H | | N | | Cl | | F | | S | |
|---|---|---|---|---|---|---|---|---|---|---|---|---|---|---|
| 37 | $C_{19}H_{26}N_2OS$ | 330,493 | 69,051 | 69,00 | 7,93 | 7,98 | 8,48 | 8,45 | | | | | 9,70 | |
| 38 | $C_{18}H_{25}N_3OS$ | 331,482 | 65,22 | 65,15 | 7,60 | 7,65 | 12,68 | 12,62 | | | | | 9,67 | |
| 39 | $C_{18}H_{25}N_3S_2$ | 347,549 | 62,21 | 62,10 | 7,25 | 7,31 | 12,09 | 12,00 | | | | | 18,45 | |
| 40 | $C_{20}H_{28}N_2OS$ | 344,520 | 69,73 | 69,78 | 8,19 | 8,24 | 8,13 | 8,08 | | | | | 9,31 | |
| 41 | $C_{19}H_{25}ClN_2OS$ | 364,939 | 62,53 | 62,51 | 6,91 | 7,00 | 7,68 | 7,71 | 9,72 | | | | 8,79 | |
| 42 | $C_{18}H_{24}ClN_3OS$ | 365,927 | 59,08 | 59,13 | 6,61 | 6,55 | 11,48 | 11,51 | 9,67 | 9,75 | | | 8,76 | |
| 43 | $C_{19}H_{25}ClN_2OS$ | 364,939 | 62,53 | 62,50 | 6,91 | 6,96 | 7,68 | 7,65 | 9,72 | | | | 8,78 | |
| 44 | $C_{18}H_{24}ClN_3OS$ | 365,927 | 59,08 | 69,11 | 6,61 | 6,60 | 11,48 | 11,41 | 9,69 | | | | 8,76 | |
| 45 | $C_{31}H_{41}ClN_2OS_2$ | 557,263 | 66,82 | 66,89 | 7,42 | 7,46 | 5,03 | 5,10 | 6,36 | | | | 11,51 | |

**Fortsetzung Tabelle 2**

| Beispiel Nr. | Summenformel | Molmasse | Elementaranalyse berechnet/gefunden C | | H | | N | | Cl | | F | | S | |
|---|---|---|---|---|---|---|---|---|---|---|---|---|---|---|
| 46 | $C_{20}H_{27}N_3O$ | 325,453 | 73,81 | 73,88 | 8,36 | 8,40 | 12,91 | 12,93 | | | | | | |
| 47 | $C_{19}H_{26}N_2O_2$ | 314,426 | 72,58 | 72,46 | 8,33 | 8,38 | 8,91 | 8,85 | | | | | | |
| 48 | $C_{18}H_{25}N_3OS$ | 331,482 | 65,22 | 65,19 | 7,60 | 7,68 | 12,68 | 12,73 | | | | | 9,67 | |

Als Beispiel für die hohe lokale in vivo Wirksamkeit der erfindungsgemäßen Verbindungen werden Behandlungsergebnisse an Experimenten mit Trichophyton mentagrophytes infizierten Labortieren angeführt.

Zur Feststellung der lokalen Wirksamkeit wurde jeweils ein 450 - 500 g schweres Meerschweinchen (Stamm Pirbright white) mit $1{,}5 \times 10^4$ Konidien/Tier in die Epidermis verteilt auf 6 Infektionspunkte, infiziert.

Die Behandlung der Tiere erfolgte dermal ab dem 3. Tag nach der Infektion an 5 aufeinander folgenden Tagen durch Aufbringen einer 0,1 %igen Präparatlösung auf 6 Infektionsstellen der Rückenseite.

Neben den mit den erfindungsgemäßen Substanzen behandelten Tieren wurde ein Tier mit der

Referenzsubstanz Bifonazol behandelt, ein Tier blieb nach der Infektion unbehandelt.

Wie in Tabelle ersichtlich, zeigten die erfindungsgemäßen Verbindungen eine deutlich stärkere Reduzierung der Mykosendurchmesser als das Standardpräparat Bifonazol, d.h. der antimykotische Effekt der erfindungsgemäßen Verbindungen war demjenigen von Bifonazol bis zu 187 % überlegen.

**Tabelle 3**

| Konzentration | Präparat Beisp. Nr. | Mykosen (Durchmesser in mm) Einzelwerte (n = 6) | | | | | | Mittelwert (Standardabweich.) | Reduktion in % |
|---|---|---|---|---|---|---|---|---|---|
| Dermal 5 x 0,1 % | 2 | 3 | 3 | 3 | 3 | 4 | 4 | 3,3 (0,5) | 139,3 |
| | 5 | 0 | 2 | 3 | 3 | 4 | 4 | 2,6 (1,5) | 176,9 |
| | 15 | 0 | 0 | 2 | 2 | 3 | 3 | 1,6 (1,3) | 287,5 |
| | 40 | 0 | 2 | 3 | 3 | 4 | 5 | 2,8 (1,7) | 164,2 |
| | 46 | 0 | 2 | 3 | 4 | 4 | 4 | 2,8 (1,6) | 164,2 |
| | 47 | 3 | 3 | 3 | 4 | 4 | 5 | 3,6 (0,8) | 127,7 |
| Dermal 5 x 0,1 % | Bifonazol | 3 | 4 | 4 | 5 | 6 | 6 | 4,6 (1,5) | 100,0 |
| Kontrolle infiziert unbehandelt | - | 12 | 12 | 13 | 15 | 17 | 19 | 14,6 (2,8) | - |

n = Anzahl Meßwerte

Als Beispiel für die Hemmung der pathogenen Phase dimorpher Hefezellen werden Ergebnisse eines in-vitro-Enzymtests angeführt, in welchen die prozentuale Hemmung freigesetzter Exoenzyme, insbesondere freigesetzter Lysophospholipase (Phospholipase B), als Maß für die Wirksamkeit bestimmt wird.

Zur Feststellung der Enzymhemmung wurde eine Suspension von Candida albicans Blastokonidien (Stamm 200/175), wobei die Keimdichte photometrisch auf eine Extinktion von 0,5 (500 nm) eingestellt war, mit Präparatelösung bzw. zur Kontrolle mit Lösungsmittellösung vermischt und zwar

a) 100 µl Präparatelösung (Suspension)
+ 900 µl Keimsuspension
b) 100 µl Lösungsmittel
+ 900 µl Keimsuspension

Jeweils 5 µl der 30 min bei 21 °C inkubierten Blastokonidiensuspension wurden auf eine Agarplatte (Sabourand Agar mit Zusatz von 8 % Eigelb, 1 M NaCl, 5 mM $CaCl_2$) aufgetropft.

Die so beimpfte Platte wurde 3 Tage bei 37 °C bebrütet.

Die Auswertung erfolgte derart, daß

1. der Durchmesser (mm) der Candida albicans-Kolonie (behandelt und unbehandelt) sowie
2. der Gesamtdurchmesser von Kolonie und Trübungshof, welcher durch Exoenzyme verursacht wurde (behandelt und unbehandelt) bestimmt wurde.

Aus der Bestimmung des Quotienten von Präparate- und Kontrollgruppe ergab sich ein Wert, der als Maß für die Enzymaktivität anzusehen war.

Wie aus Tabelle 4 ersichtlich, hemmen die erfindungsgemäßen Verbindungen die freigesetzten Exoenzyme wesentlich stärker als Propranolol.

Propranolol wird in der Literatur (Pappu A.S. et al. in Biochem. Pharmacol. 34, 521-24, 1985) als wirksamste Substanz beschrieben, die gegenüber Phospholipase aus Leberzellen in einem entsprechenden in-vitro-Test Hemmwirkung zeigte.

Der Stand der Technik wird durch die erfindungsgemäßen Verbindungen überraschenderweise deutlich übertroffen.

Tabelle 4

Prozentuale Hemmung der freigesetzten Exoenzyme von Candida albicans in vitro

| Präparat Beispiel Nr. | Präparat-Konzentration 100 µg/ml | 50 µg/ml | 10 µg/ml |
|---|---|---|---|
| 2 | 100 | 100 | 41,2 |
| 15 | 100 | 100 | 72,2 |
| 42 | 100 | 100 | 50,0 |
| Propranolol | 30 | 10 | 0 |
| Lösungsmittel Kontrolle | 0 | 0 | 0 |

## Ansprüche

**1.** Aryl-alkyl-imidazol der Formel I

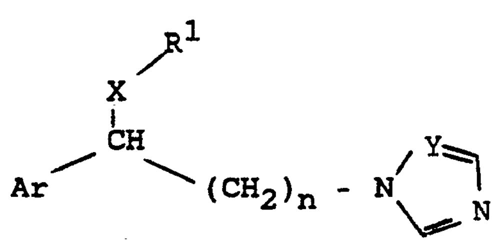

(I),

worin bedeuten

Ar Phenyl, Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Furyl, Pyridyl, Pyrimidyl, Oxazolyl, Isoxazolyl, Pyridazyl, Pyrazyl, Pyrazolyl, Benzofuryl, Benzothienyl, Benzothiazolyl, Benzoimidazolyl,

wobei die genannten Ringsysteme unsubstituiert oder mit 1 bis 3 Substituenten, die gleich oder verschieden sind, substituiert sind, welche F, Cl, Br, I, $(C_1-C_8)$-Alkyl (geradkettig oder verzweigt), $(C_nF_{2n+1})$ mit n = 1-8, $C_2F_4$, Monofluorbutyl, $CCl_3$, $(C_3-C_8)$-Cycloalkyl, $(C_1-C_8)$-Alkoxy (geradkettig oder verzweigt), $(C_3-C_8)$-Cycloalkyl-$(C_1-C_4)$-Alkyl, $(C_1-C_8)$-Alkylthio, $(C_1-C_8)$-Alkylsulfinyl- und -sulfonyl, $NO_2$, CN, $CF_3$ sind;

Y CH oder N

n 1 oder 2

X O, S, SO, $SO_2$, $CH_2$

$R^1$ mehrfach ungesättigtes $(C_5-C_{20})$-Alkenyl geradkettig oder verzweigt, mehrfach ungesättigtes $(C_5-C_{20})$-Alkinyl geradkettig oder verzweigt, mehrfach ungesättigtes $(C_5-C_{20})$-Alken-in-yl, geradkettig oder verzweigt,

und ihre Salze mit pharmakologisch akzeptablen Säuren.

2. Verbindung der Formel I nach Anspruch 1, dadurch gekennzeichnet, daß die Substituenten und Indices folgende Bedeutung haben

Ar Phenyl, Biphenylyl, 1,2,3,4-Tetrahydronaphthyl, Naphthyl, Thienyl, Indanyl (jeweils unsubstituiert oder im aromatischen Ring mit ein oder zwei Substituenten, die gleich oder verschieden sind und jeweils F, Cl, Br, $CF_3$, $(C_1-C_4)$-Alkyl oder $(C_1-C_4)$-Alkoxy bedeuten, substituiert),

Y CH oder N

n 1 oder 2

X O, S, SO, $SO_2$, $CH_2$,

$R^1$ mehrfach ungesättigtes $(C_5-C_{20})$-Alkenyl, geradkettig oder verzweigt, mehrfach ungesättigtes $(C_5-C_{20})$-Alkinyl, geradkettig oder verzweigt, mehrfach ungesättigtes $(C_5-C_{20})$-Alken-in-yl, geradkettig oder verzweigt,

und ihre Salze mit physiologisch verträglichen Säuren.

3. Verbindung der Formel I nach Anspruch 1, dadurch gegekennzeichnet, daß Substituenten und Indices folgende Bedeutung haben

Ar Phenyl, Biphenylyl, Naphthyl, Thienyl, Indanyl (jeweils unsubstituiert oder im aromatischen Ring mit ein oder zwei Substituenten, die gleich oder verschieden sind und jeweils F, Cl, $CF_3$, $(C_1-C_4)$-Alkyl oder $(C_1-C_4)$-Alkoxy bedeuten, substituiert),

Y CH oder N

n 1 oder 2

X O, S, SO, $SO_2$,

$R^1$ mehrfach ungesättigtes $(C_5-C_{20})$-Alkenyl geradkettig oder verzweigt, mehrfach ungesättigtes $(C_5-C_{20})$-Alkinyl geradkettig oder verzweigt, mehrfach ungesättigtes $(C_5-C_{20})$-Alken-in-yl, geradkettig oder verzweigt,

und ihre Salze mit physiologisch verträglichen Säuren.

4. Verbindung der Formel I nach Anspruch 1, dadurch gekennzeichnet, daß Substituenten und Indices folgende Bedeutung haben

Ar Phenyl, Biphenylyl, Indanyl, Thienyl, Naphthyl, (jeweils unsubstituiert oder im aromatischen Ring mit ein oder zwei Substituenten, die gleich oder verschieden sind und F-oder Cl-Atome, $CF_3$, Methyl und Methoxy bedeuten, substituiert)

Y CH oder N

n 1

X O, S, SO, $SO_2$,

$R^1$ mehrfach ungesättigtes $(C_5-C_{20})$-Alkenyl, geradkettig oder verzweigt, mehrfach ungesättigtes $(C_5-C_{20})$-Alkinyl, geradkettig oder verzweigt, mehrfach ungesättigtes $(C_5-C_{20})$-Alken-in-yl, geradkettig oder verzweigt,

und ihre Salze mit physiologisch verträglichen Säuren.

5. Verfahren zum Herstellen einer Verbindung der Formel I nach Anspruch 1, dadurch gekennzeichnet, daß man

a) eine Verbindung der Formel II

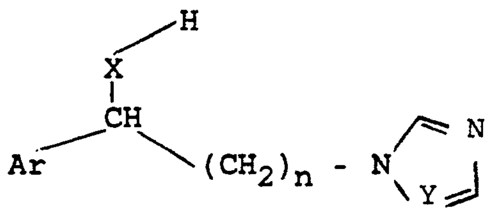

(II),

in welcher Ar, X, n und Y die in Anspruch 1 angegebenen Bedeutungen haben, mit einer Verbindung der Formel (III)

$$R^1 - Nu \qquad (III)$$

umsetzt, worin $R^1$ die unter Anspruch 1 angegebene Bedeutung hat und Nu Fluor, Chlor, Brom, Jod, Trifluormethansulfonat, p.Toluolsulfonat, Methansulfonat oder ein anderes üblicherweise verwendetes Nucleofug bedeutet,
und eine so erhaltene Verbindung der Formel (I) in Form der freien Base oder eines Säureadditionsalzes oder eines hydrolysierbaren Derivats isoliert,
oder daß man

b) Verbindungen der Formel V

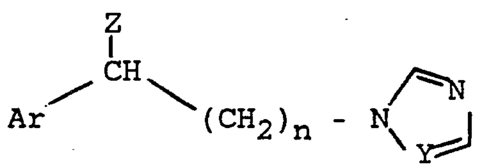

(V),

in denen Z Fluor, Chlor, Brom, Jod, Trifluormethansulfonat, p-Toluolmethansulfonat, Methansulfonat oder ein anderes üblicherweise verwendetes Nucleofug bedeutet,
mit Verbindungen der Formel VI

$$R^1 - X\,H \qquad (VI)$$

in denen $R^1$ und X die in Anspruch 1 angegebene Bedeutung haben,
umsetzt.

6. Verwendung einer Verbindung der Formel I nach Anspruch 1 zur Herstellung eines Antimykotikums.

7. Verwendung einer Verbindung der Formel I nach Anspruch 1 als Antimykotikum.

8. Antimykotische Zubereitung, gekennzeichnet durch einen wirksamen Gehalt einer Verbindung der Formel I nach Anspruch 1 und pharmazeutisch annehmbaren Zusatzstoffen.

9. Verwendung einer Verbindung der Formel I nach Anspruch 1 als Wachstumshemmer der pathogenen Phase dimorpher Hefen.